# EUROPEAN PATENT APPLICATION

(11) **EP 4 844 067 A1**
(43) Date of publication of application: **25.11.2026**
(21) Application number: 25741899.6
(22) Date of filing: 10.01.2025
(51) Int. Cl.: C12N 15/57, C12N 9/54, C12P 7/625

(54) **METHOD FOR PRODUCING BIOPOLYMER**

(30) Priority: 15.01.2024 JP 2024003764; 25.04.2024 JP 2024071822
(71) Applicant: Kao Corporation, Tokyo 103-8210 (JP)
(72) Inventor: HIOKI, Takahiro, Tokyo 103-8210 (JP); OSAMURA, Tatsuya, Tokyo 103-8210 (JP); TAKAHASHI, Fumikazu, Tokyo 103-8210 (JP); KAWAHARA, Akihito, Tokyo 103-8210 (JP)
(74) Representative: Vossius & Partner Patentanwälte Rechtsanwälte mbB
(86) International application number: PCT/JP2025/000651
(87) International publication number: WO 2025/154673

(57) **Abstract**

Provided is a method for enhancing the purity of a biopolymer in producing the biopolymer using a microorganism. The present invention provides a method for producing a biopolymer using a microorganism, comprising using at least one protease selected from the group consisting of the following a) and b) in a purification step: a) a protease consisting of an amino acid sequence having at least 90% identity to SEQ ID NO: 1 or 4, and b) a protease consisting of an amino acid sequence having at least 90% identity to SEQ ID NO: 5 or 8.

## Description

### Field of the Invention

The present invention relates to a method for producing a biopolymer.

### Background of the Invention

Biopolymers using biomass as starting materials are utilized as petrochemical plastic alternatives in various applications. Many biopolymers have high biodegradability, and demand for such biopolymers has been expanding from the viewpoint of environmental protection in recent years. Typical examples of the biopolymer include starch, polylactic acid (PLA), and polyhydroxyalkanoate (PHA).

PHA, one of the biopolymers, can be produced by microbial fermentation. Since PHA is accumulated as granules in cells, PHA production requires a downstream step for PHA recovery. This downstream step is very expensive in the PHA production and is responsible for difficulty in commercializing PHA. Approaches using an organic solvent such as chloroform are widely used in PHA recovery. Such approaches, however, require a large amount of a toxic and volatile solvent and also require a lot of energy, and thus, their environmental burdens are of concern (Non Patent Literature 1).

Meanwhile, approaches using an enzyme have been studied since a long time ago in order to recover PHA without the use of an organic solvent. Enzymatic degradation of cellular constituents such as cell walls, proteins, sugars, nucleic acids, and lipids can produce effects such as promotion of PHA release through the weakening of cells and improvement of a PHA purity through the degradation of impurities. Because of high substrate specificity and mild reaction conditions, enzymes have the advantages in that damage on PHA is small, necessary energy or effluent load is small, and influence on the environment is small.

Among the enzymes, particularly, proteases are widely used in PHA recovery, and Alcalase, Esperase, alkaline protease, trypsin, or the like is suitably used (Non Patent Literatures 1 to 3 and Patent Literatures 1 and 2). Since the proteases are often used in the presence of a surfactant or under alkaline conditions, microorganism-derived proteases which exhibit high activity under these conditions and are inexpensive are particularly utilized. However, industrial proteases used so far might not be sufficiently optimized for PHA recovery, and there is no previous report on any mutant protease improved in performance in PHA recovery. A major challenge to PHA recovery using an enzyme is cost of the enzyme (Patent Literature 3). Therefore, proteases having higher performance than conventional ones are required for PHA recovery in order to decrease a necessary amount of the enzyme. Furthermore, such proteases having high performance are required for the purpose of further improving the purity of PHA.

(Patent Literature 1) CN 1070534 B
(Patent Literature 2) WO 2023/021878
(Patent Literature 3) CN 109504715 A
(Non Patent Literature 1) Gonzalez, K., et al. Current microbiology 78 (2021): 1-10
(Non Patent Literature 2) Neves, A. and Mueller, J. Biotechnology progress 28.6 (2012): 1575-1580
(Non Patent Literature 3) Kapritchkoff, F.M., et al. Journal of biotechnology 122.4 (2006): 453-462

### Summary of the Invention

The present invention relates to the following 1) to 5).
1) A method for producing a biopolymer using a microorganism having the ability to produce the biopolymer, comprising using at least one protease selected from the group consisting of the following a) and b) in a biopolymer purification step:
   a) a protease consisting of an amino acid sequence having at least 90% identity to SEQ ID NO: 1 or 4, and
   b) a protease consisting of an amino acid sequence having at least 90% identity to SEQ ID NO: 5 or 8.
2) A method for purifying a biopolymer, comprising using at least one protease selected from the group consisting of the above a) and b) in a method for producing a biopolymer using a microorganism having the ability to produce the biopolymer.
3) An enzyme composition for biopolymer purification, comprising at least one protease selected from the group consisting of the above a) and b).
4) Use of at least one protease selected from the group consisting of the above a) and b) for the production of an enzyme composition for biopolymer purification.
5) Use of at least one protease selected from the group consisting of the above a) and b) as an enzyme for biopolymer purification.

### Detailed Description of the Invention

All patent literatures, non patent literatures, and other publications cited herein are incorporated herein by reference in their entirety.

As used herein, the "protease" means an enzyme group (EC3.4) having protease activity of hydrolyzing a peptide bond in a protein molecule to form a peptide or an amino acid. The protease activity can be measured by a method known in the art.

In the present specification, the identity of an amino acid sequence or a nucleotide sequence is calculated in accordance with Lipman-Pearson method (Science, 1985, 227: 1435-1441). Specifically, the identity is calculated by analysis using the Search homology program of genetic information processing software GENETYX Ver. 12 with Unit size to compare (ktup) as 2.

As used herein, the "at least 90% identity" regarding an amino acid sequence or a nucleotide sequence refers to 90% or higher, preferably 95% or higher, more preferably 97% or higher, further more preferably 98% or higher, further more preferably 99% or higher identity.

As used herein, the "position corresponding" on an amino acid sequence or a nucleotide sequence can be determined by aligning a target sequence and a reference sequence (e.g., the amino acid sequence represented by SEQ ID NO: 1) so as to give the maximum homology. The alignment of amino acid sequences or nucleotide sequences can be carried out using a known algorithm, and the procedure thereof is known to those skilled in the art. For example, the alignment can be performed by using Clustal W multiple alignment program (Thompson, J.D., et al., 1994, Nucleic Acids Res., 22: 4673-4680) with the default set. Alternatively, Clustal W2 or Clustal omega, which are revised editions of Clustal W, can also be used. Clustal W, Clustal W2, and Clustal omega can be used on the website of, for example, the Clustal [www.clustal.org] operated by University College Dublin, the European Bioinformatics Institute: EBI [www.ebi.ac.uk/index.html], or the DNA data bank of Japan (DDBJ [www.ddbj.nig.ac.jp/searches-j.html]) operated by the National Institute of Genetics. A position in a target sequence aligned at any position in a reference sequence by the alignment mentioned above is regarded as the "position corresponding" to the position.

Those skilled in the art can further finely adjust and optimize the alignment of the amino acid sequence obtained above. The optimum alignment is preferably determined by considering the similarity of amino acid sequences, the frequency of gaps to be inserted, and so on. Here, the similarity of amino acid sequences refers to, when two amino acid sequences are aligned, the ratio (%) of the number of positions where the same or similar amino acid residues are present in both sequences to the number of the full-length amino acid residues. The similar amino acid residues mean that the amino acid residues have similar properties in the polarity or electric charge to each other and cause so-called conservative substitution among 20 amino acids constituting a protein. Groups consisting of such similar amino acid residues are well known to those skilled in the art, and examples thereof include, but are not limited to, arginine and lysine or glutamine; glutamic acid and aspartic acid or glutamine; serine and threonine or alanine; glutamine and asparagine or arginine; and leucine and isoleucine.

As used herein, the "amino acid residue" means 20 amino acid residues constituting a protein: alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y), and valine (Val or V).

In the present specification, an amino acid position and mutant are designated using the accepted IUPAC one-letter amino acid abbreviation as follows.

An amino acid at a predetermined position is designated by [amino acid, position]. For example, serine at position 16 is indicated in "S16".

The "substitution" of an amino acid is designated by [original amino acid, position, substituted amino acid]. For example, the substitution of serine at position 16 with valine is indicated in "S16V".

A mutant including a plurality of variations is indicated by an addition symbol ("+"). For example, "S16V + T65P" represents the substitution of serine at position 16 with valine and the substitution of threonine at position 65 with proline.

As used herein, the "upstream" and the "downstream" regarding a gene refer to the upstream and the downstream of the gene in the transcription direction. For example, the "gene placed downstream of a promoter" means that the gene is present on the 3' side of the promoter in a DNA sense strand, and the upstream of a gene means a region on the 5' side of the gene in a DNA sense strand.

As used herein, the "parent" polypeptide of a given mutant polypeptide refers to a polypeptide that brings about the mutant polypeptide by a predetermined mutation of an amino acid residue thereof. In other words, the "parent" polypeptide is a polypeptide before the mutation is introduced into the mutant polypeptide.

The present invention relates to a method for enhancing the purity of a biopolymer in producing the biopolymer using a microorganism having the ability to produce the biopolymer.

The present inventors have found a protease having a higher effect of reducing the amount of residues than that of conventional proteases commonly used in a biopolymer purification step, when the protease is used in the biopolymer purification step in producing a biopolymer using a microorganism having the ability to produce the biopolymer, and used the protease as a parent protease to give a protease mutant having a higher effect of reducing the amount of residues than that of the parent protease.

The method of the present invention can improve the purity of a biopolymer in a method for producing a biopolymer using a microorganism having the ability to produce the biopolymer.

The protease for use in the method for producing a biopolymer and the method for purifying a biopolymer according to the present invention (referred to as the "protease of the present invention") is at least one polypeptide having proteolytic (protease) activity, the polypeptide being selected from the group consisting of the following a) and b):
a) a protease consisting of an amino acid sequence having at least 90% identity to SEQ ID NO: 1 or 4, and
b) a protease consisting of an amino acid sequence having at least 90% identity to SEQ ID NO: 5 or 8.

The protease of the present invention is preferably at least one mutant polypeptide having proteolytic (protease) activity, the mutant polypeptide being selected from the group consisting of a') and b') given below from the viewpoint of the effect of reducing the amount of residues. These proteases may be used singly or in combinations of two or more thereof as the protease of the present invention.
a') A protease which consists of an amino acid sequence having at least 90% identity to SEQ ID NO: 1 or 4 and has at least one amino acid residue selected from the group consisting of valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1, and
b') a protease which consists of an amino acid sequence having at least 90% identity to SEQ ID NO: 5 or 8 and has at least one amino acid residue selected from the group consisting of glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.

The protease consisting of the amino acid sequence of SEQ ID NO: 1 is a mature enzyme of alkaline protease KP43 derived from *Bacillus* sp. KSM-KP43 (FERM BP-6532), and is obtained by cleaving and removing a prepro sequence through processing from an alkaline protease precursor consisting of the amino acid sequence of SEQ ID NO: 2 and comprising the prepro sequence and a mature enzyme region of the alkaline protease KP43 (JP-A-2021-97605).

The protease consisting of the amino acid sequence of SEQ ID NO: 4 is a mature enzyme of an alkaline protease KP43 mutant consisting of an amino acid sequence derived from the amino acid sequence of SEQ ID NO: 1 by the substitution of amino acid residue at position 16 with valine, the substitution of amino acid residue at position 65 with proline, the substitution of amino acid residue at position 83 with alanine, the substitution of amino acid residue at position 273 with isoleucine, the substitution of amino acid residue at position 359 with serine, the substitution of amino acid residue at position 387 with alanine, the substitution of amino acid residue at position 132 with threonine, the substitution of amino acid residue at position 166 with glycine, the substitution of amino acid residue at position 167 with valine, the substitution of amino acid residue at position 195 with glutamine, the substitution of amino acid residue at position 294 with threonine, and the substitution of amino acid residue at position 369 with asparagine. The amino acid sequence of SEQ ID NO: 1 and the amino acid sequence of SEQ ID NO: 4 have approximately 97% identity.

One example of the parent protease of the protease a') of the present invention includes a protease consisting of an amino acid sequence having at least 90% identity to the amino acid sequence represented by SEQ ID NO: 1 of the protease a) of the present invention. Another example of the parent protease of the protease a') of the present invention includes a protease consisting of an amino acid sequence having at least 90% identity to the amino acid sequence represented by SEQ ID NO: 4 of the protease a) of the present invention. The protease consisting of the amino acid sequence of SEQ ID NO: 4 is also the protease a) of the present invention, is also the parent protease of the protease a') of the present invention, and also the protease a') of the present invention, the parent protease of which is the protease consisting of the amino acid sequence of SEQ ID NO: 1.

The protease a') of the present invention is a protease which consists of an amino acid sequence having at least 90% identity to the amino acid sequence of SEQ ID NO: 1 or 4 and has at least one of 12 amino acid residues: valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1, preferably 3 or more, more preferably 6 or more, further more preferably 10 or more, further more preferably all, of the 12 amino acid residues.

The protease consisting of the amino acid sequence of SEQ ID NO: 5 is a mature enzyme of alkaline protease K16 derived from *Bacillus* sp. KSM-K16 (FERM BP-3376), and is obtained by cleaving and removing a prepro sequence through processing from an alkaline protease precursor consisting of the amino acid sequence of SEQ ID NO: 6 and comprising the prepro sequence and a mature enzyme region of the alkaline protease K16 (Kobayashi, T., et al. Applied Microbiology and Biotechnology 43 (1995): 473-481).

The protease consisting of the amino acid sequence of SEQ ID NO: 8 is a mature enzyme of an alkaline protease K16 mutant consisting of an amino acid sequence derived from the amino acid sequence of SEQ ID NO: 5 by the substitution of amino acid residue at position 9 with glutamic acid, the substitution of amino acid residue at position 253 with aspartic acid, the substitution of amino acid residue at position 255 with tryptophan, and the substitution of amino acid residue at position 256 with glutamic acid. The amino acid sequence of SEQ ID NO: 5 and the amino acid sequence of SEQ ID NO: 8 have approximately 99% identity.

One example of the parent protease of the protease b') of the present invention includes a protease consisting of an amino acid sequence having at least 90% identity to the amino acid sequence represented by SEQ ID NO: 5. Another example of the parent protease of the protease b') of the present invention includes a protease consisting of an amino acid sequence having at least 90% identity to the amino acid sequence represented by SEQ ID NO: 8. The protease consisting of the amino acid sequence of SEQ ID NO: 8 is also the protease b) of the present invention, is also the parent protease of the protease b') of the present invention, and also the protease b') of the present invention, the parent protease of which is the protease consisting of the amino acid sequence of SEQ ID NO: 5.

The protease b') of the present invention is a protease which consists of an amino acid sequence having at least 90% identity to the amino acid sequence of SEQ ID NO: 5 or 8 and has at least one of 4 amino acid residues: glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5, preferably 2 or more, more preferably 3 or more, further more preferably all, of the 4 amino acid residues.

The protease of the present invention may be microbiologically produced or may be chemically synthesized. In the microbiological production of the protease of the present invention, the protease of the present invention is preferably expressed as a protease precursor (proprotein) comprising a pro sequence and a mature enzyme region or a protease precursor (preproprotein) comprising a signal sequence (pre sequence), a pro sequence, and a mature enzyme region.

The pro sequence of the protease is involved in the folding of the protease. In the protease precursor comprising the pro sequence, the pro sequence is positioned on the N-terminal side of the mature enzyme region of the protease precursor. The pro sequence acts as an intramolecular chaperonin and is an essential region for the mature protease to assume a correct three-dimensional structure before and after passing through a cell membrane. The pro sequence is finally cleaved by the mature protease and degraded into smaller peptides. Examples of the pro sequence include a sequence consisting of an amino acid sequence having at least 90% identity to an amino acid sequence from positions 32 to 206 of SEQ ID NO: 2, and a sequence consisting of an amino acid sequence having at least 90% identity to an amino acid sequence from positions 28 to 111 of SEQ ID NO: 6 and include a pro sequence of the alkaline protease KP43 and a pro sequence of the alkaline protease K16 as mentioned above.

In the preproprotein of the protease, the signal sequence is positioned on the N-terminal side of the pro sequence. The signal sequence is involved in extracellular secretion of the protease. When the preproprotein passes through a cell membrane, the signal sequence is cleaved by signal peptidase to form a proprotein. Examples of the signal sequence include a sequence consisting of an amino acid sequence having at least 90% identity to an amino acid sequence from positions 1 to 31 of SEQ ID NO: 2, and a sequence consisting of an amino acid sequence having at least 90% identity to an amino acid sequence from positions 1 to 27 of SEQ ID NO: 6 and include a signal sequence of the alkaline protease KP43 and a signal sequence of the alkaline protease K16 as mentioned above.

Accordingly, the protease precursor of the protease a) of the present invention can be a polypeptide which comprises a pro sequence and a mature enzyme region and consists of an amino acid sequence having at least 90% identity to an amino acid sequence from positions 32 to 640 of SEQ ID NO: 2 or to an amino acid sequence from positions 32 to 206 of SEQ ID NO: 2 and the amino acid sequence of SEQ ID NO: 4. Alternatively, the protease precursor of the protease a) of the present invention can be a polypeptide which comprises a signal sequence, a pro sequence and a mature enzyme region and consists of an amino acid sequence having at least 90% identity to the amino acid sequence of SEQ ID NO: 2 or to an amino acid sequence from positions 1 to 206 of SEQ ID NO: 2 and the amino acid sequence of SEQ ID NO: 4.

The protease precursor of the protease a') of the present invention can be a polypeptide which comprises a pro sequence and a mature enzyme region, consists of an amino acid sequence having at least 90% identity to an amino acid sequence from positions 32 to 640 of SEQ ID NO: 2 or to an amino acid sequence from positions 32 to 206 of SEQ ID NO: 2 and the amino acid sequence of SEQ ID NO: 4, and has at least one amino acid residue selected from the group consisting of valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1. Alternatively, the protease precursor of the protease a') of the present invention can be a polypeptide which comprises a signal sequence, a pro sequence and a mature enzyme region, consists of an amino acid sequence having at least 90% identity to the amino acid sequence of SEQ ID NO: 2 or to an amino acid sequence from positions 1 to 206 of SEQ ID NO: 2 and the amino acid sequence of SEQ ID NO: 4, and has at least one amino acid residue selected from the group consisting of valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1.

The protease precursor of the protease b) of the present invention can be a polypeptide which comprises a pro sequence and a mature enzyme region and consists of an amino acid sequence having at least 90% identity to an amino acid sequence from positions 28 to 380 of SEQ ID NO: 6 or to an amino acid sequence from positions 28 to 111 of SEQ ID NO: 6 and the amino acid sequence of SEQ ID NO: 8. Alternatively, the protease precursor of the protease b) of the present invention can be a polypeptide which comprises a signal sequence, a pro sequence and a mature enzyme region and consists of an amino acid sequence having at least 90% identity to the amino acid sequence of SEQ ID NO: 6 or to an amino acid sequence from positions 1 to 111 of SEQ ID NO: 6 and the amino acid sequence of SEQ ID NO: 8.

The protease precursor of the protease b') of the present invention can be a polypeptide which comprises a pro sequence and a mature enzyme region, consists of an amino acid sequence having at least 90% identity to an amino acid sequence from positions 28 to 380 of SEQ ID NO: 6 or to an amino acid sequence from positions 28 to 111 of SEQ ID NO: 6 and the amino acid sequence of SEQ ID NO: 8, and has at least one amino acid residue selected from the group consisting of glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5. Alternatively, the protease precursor of the protease b') of the present invention can be a polypeptide which comprises a signal sequence, a pro sequence and a mature enzyme region, consists of an amino acid sequence having at least 90% identity to the amino acid sequence of SEQ ID NO: 6 or to an amino acid sequence from positions 1 to 111 of SEQ ID NO: 6 and the amino acid sequence of SEQ ID NO: 8, and has at least one amino acid residue selected from the group consisting of glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.

The protease precursor can be produced by expressing a polypeptide from a polynucleotide encoding the protease precursor. The polynucleotide can be prepared from a microorganism which produces the protease precursor of interest by extracting genomic DNA by a routine method or extracting RNA and synthesizing cDNA therefrom by reverse transcription. Alternatively, a corresponding nucleotide sequence may be chemically synthesized on the basis of the amino acid sequence of the protease precursor of interest and used as a polynucleotide encoding the protease precursor of interest.

Alternatively, the protease precursor can be produced using various mutagenesis techniques known in the art. For example, the protease precursor can be produced by mutating a polynucleotide encoding the amino acid residue to be substituted in a parent protease gene (reference protease gene) encoding the reference amino acid sequence into a polynucleotide encoding an amino acid residue after substitution, and further expressing the mutant polypeptide from the mutant gene.

Various mutagenesis techniques known in the art can be used as approaches for mutating an amino acid of the protease precursor. For example, a polynucleotide (hereinafter, also referred to as a parent gene) encoding an amino acid sequence (parent protease sequence) to be mutated can be changed into a polynucleotide (hereinafter, also referred to as a mutant gene) encoding a mutant amino acid sequence so that a protease mutant having the mutation of interest is expressed from the mutant gene.

Basically, a mutation can be introduced into the parent gene using various site-directed mutagenesis methods known to those skilled in the art. The site-directed mutagenesis can be performed by an arbitrary method such as inverse PCR or annealing. Commercially available site-directed mutagenesis kits (e.g., QuickChange II Site-Directed Mutagenesis Kit and QuickChange Multi Site-Directed Mutagenesis Kit of Stratagene) can also be used.

Site-directed mutagenesis into the parent gene can be most generally performed using a mutation primer including a nucleotide mutation to be introduced. The mutation primer may be designed so as to anneal with a region comprising a nucleotide sequence encoding an amino acid residue to be mutated in the parent gene and to comprise a nucleotide sequence having a nucleotide sequence (codon) encoding an amino acid residue after the mutation instead of the nucleotide sequence (codon) encoding the amino acid residue to be mutated. Nucleotide sequences (codons) encoding amino acid residues before and after mutation can be appropriately recognized and selected by those skilled in the art on the basis of ordinary textbooks and the like. Alternatively, a method of linking DNA fragments into one by SOE (splicing by overlap extension)-PCR (Gene, 1989, 77 (1): 61-68), the DNA fragments being separately obtained by amplifying the upstream and downstream sides of a mutation site using two complementary primers including the nucleotide mutation to be introduced can be used for site-directed mutagenesis.

Template DNA comprising the parent gene can be prepared from, for example, a strain which produces the parent protease (e.g., *Bacillus* sp. KSM-KP43 or *Bacillus* sp. KSM-K16) by extracting genomic DNA by a routine method or extracting RNA and synthesizing cDNA therefrom by reverse transcription. Alternatively, a corresponding nucleotide sequence may be chemically synthesized on the basis of the amino acid sequence of the parent protease and used as template DNA. The parent gene may be codon-optimized, if necessary, depending on the type of a transformant which is allowed to express the protease precursor. Information on codons to be used by various organisms is available from the Codon Usage Database ([www.kazusa.or.jp/codon/]) .

The parent gene preferably encodes a proprotein of the protease comprising a pro sequence and a mature enzyme region or a preproprotein of the protease comprising a signal sequence, a pro sequence, and a mature enzyme region. Examples of the parent gene of the protease a') of the present invention include a polynucleotide encoding an amino acid sequence from positions 32 to 640 of SEQ ID NO: 2, a polynucleotide encoding the amino acid sequence of SEQ ID NO: 2, and a polynucleotide consisting of the nucleotide sequence of SEQ ID NO: 3. Examples of the parent gene of the protease b') of the present invention include a polynucleotide encoding an amino acid sequence from positions 28 to 380 of SEQ ID NO: 6, a polynucleotide encoding the amino acid sequence of SEQ ID NO: 6, and a polynucleotide consisting of the nucleotide sequence of SEQ ID NO: 7.

The polynucleotide encoding the protease precursor can comprise a single-stranded or double-stranded DNA, cDNA, RNA, or another artificial nucleic acid. The DNA, the cDNA, and the RNA may be chemically synthesized. The polynucleotide of the present invention may comprise an open reading frame (ORF) as well as the nucleotide sequence of an untranslated region (UTR). The polynucleotide may be codon-optimized depending on the type of a transformant for protease precursor production.

The obtained polynucleotide encoding the protease precursor can be incorporated into a vector. The vector can be prepared by inserting the polynucleotide into an arbitrary vector by a routine method. The type of the vector is not particularly limited and may be any vector such as a plasmid, a phage, a phagemid, a cosmid, a virus, a YAC vector, and a shuttle vector. The vector is, but not limited to, preferably a vector capable of being amplified in a bacterium, preferably a bacterium of the genus *Bacillus* (e.g., *Bacillus subtilis* or its mutant strain), more preferably an expression vector capable of inducing the expression of a transgene in a bacterium of the genus *Bacillus.* In particular, a shuttle vector, which is a vector capable of replicating in all of bacteria of the genus *Bacillus* and other organisms, can be suitably used for recombinant production of the mutant of the present invention. Preferable examples of the vector include, but are not limited to, shuttle vectors such as pHA3040SP64, pHSP64R, and pASP64 (JP-B-3492935), pHY300PLK (expression vector capable of transforming both *Escherichia coli* and *Bacillus subtilis;* Jpn J. Genet., 1985, 60: 235-243), and pAC3 (Nucleic Acids Res., 1988, 16: 8732); and plasmid vectors that can be used for the transformation of bacteria of the genus *Bacillus,* such as pUB110 (J. Bacteriol., 1978, 134: 318-329) and pTA10607 (Plasmid, 1987, 18: 8-15). Alternatively, plasmid vectors derived from *Escherichia coli* (e.g., pET22b(+), pBR322, pBR325, pUC57, pUC118, pUC119, pUC18, pUC19, and pBluescript) may be used.

For recombinant production of the protease precursor, the vector is preferably an expression vector. The expression vector may optionally comprise useful sequences including various elements essential for expression in a host, such as a transcriptional promoter, a terminator, and a ribosomal binding site; cis elements such as a polylinker and an enhancer; a polyA addition signal; a ribosomal binding sequence (SD sequence); and a selective marker gene such as a drug (e.g., ampicillin, neomycin, kanamycin, tetracycline, or chloramphenicol) resistance gene. Alternatively, the polynucleotide encoding the protease precursor may comprise the useful sequences described above.

A transformant comprising the polynucleotide encoding the protease precursor or the vector containing the polynucleotide can be obtained by introducing the polynucleotide encoding the protease precursor or the vector containing the polynucleotide into a host.

Examples of the host of the transformant include bacteria of the genus *Bacillus* such as *Bacillus subtilis,* bacteria of the genus *Clostridium,* and yeasts. Among them, a bacterium of the genus *Bacillus* is preferred, and *Bacillus subtilis* or a mutant strain thereof is more preferred. Thus, the transformant is preferably a recombinant bacterium of the genus *Bacillus,* more preferably a recombinant of *Bacillus subtilis* or a mutant strain thereof. Examples of the mutant strain of *Bacillus subtilis* include strains lacking aprX and a gene selected from the group consisting of aprE, nprB, nprE, bpr, vpr, mpr, epr, and wprA (JP-A-2006-174707).

For example, well known transformation technique such as a calcium phosphate technique, electroporation, lipofection, a particle gun technique or a PEG technique can be applied to the introduction of the polynucleotide or the vector to host cells. Examples of the method applicable to *Bacillus subtilis* or a mutant strain thereof include competent cell transformation (J Bacteriol, 1967, 93: 1925-1937), electroporation (FEMS Microbiol Lett, 1990, 55: 135-138), protoplast transformation (Mol Gen Genet, 1979, 168: 111-115), and Tris-PEG (J Bacteriol, 1983, 156: 1130-1134).

The transformant can be cultured in a proper culture medium to express a protease precursor. The expressed protease precursor becomes a mature enzyme of the protease through folding and the cleavage of the pro sequence. When the protease precursor is a preproprotein having a signal sequence, the mature enzyme is extracellularly secreted and produced.

The culture of the transformant for protease production can be performed in accordance with a general method in the art. For example, when the transformant is *Bacillus subtilis* or a mutant strain thereof, the culture medium for culture thereof contains a carbon source and an inorganic nitrogen source or an organic nitrogen source necessary for the growth of *Bacillus subtilis.* Examples of the carbon source include glucose, dextran, soluble starch, sucrose, and methanol. Examples of the inorganic nitrogen source or the organic nitrogen source include ammonium salts, nitrates, amino acids, corn steep liquor, peptone, casein, meat extracts, soybean cake, and potato extracts. The culture medium may optionally contain other nutrients, for example, inorganic salts (e.g., sodium chloride, calcium chloride, sodium dihydrogen phosphate, and magnesium chloride), vitamins, and antibiotics (e.g., tetracycline, neomycin, kanamycin, spectinomycin, and erythromycin). Culture conditions, for example, a temperature, aeration stirring conditions, pH of the culture medium, and a culture time, can be appropriately selected depending on the type and trait of the microorganism, a culture scale, and so on.

After culture, the mature enzyme of the protease is recovered from the obtained cultures by a routine method. The mature enzyme can be recovered from the cultures, for example, by recovering the cultures, performing, if necessary, microbial cell disruption treatment under ultrasonic wave or pressure, and appropriately combining filtration, centrifugation, ultrafiltration, salting-out, chromatography, and the like. When the protease precursor has a signal sequence and the mature enzyme is extracellularly secreted and produced, the mature enzyme of the protease can be recovered without disruption of cells. The degree of purification of the mature enzyme is not particularly limited. For example, a culture supernatant or a crude separation and purification product thereof can be obtained as a composition containing the mature enzyme.

The protease of the present invention, when used in a biopolymer purification step in producing a biopolymer using a microorganism having the ability to produce the biopolymer has a higher effect of reducing the amount of residues than that of Alcalase and Esperase (both from Novozymes A/S), which are conventional proteases commonly used in the purification step, and in addition, than that of the parent protease if the protease of the present invention is a protease mutant, as shown in Examples described later, and can largely contribute to improvement in the purity of the biopolymer by efficiently degrading microbial cell-derived components, other than the biopolymer, of the microorganism. Furthermore, a necessary amount of the enzyme is small, also leading to cost reduction of a method for producing a biopolymer.

Thus, the protease of the present invention is useful as an enzyme for biopolymer purification, particularly, as an enzyme for biopolymer purification for use in a purification step in a method for producing a biopolymer using a microorganism having the ability to produce the biopolymer, and is capable of serving as an active ingredient for an enzyme composition for biopolymer purification, particularly, for an enzyme composition for biopolymer purification for use in a purification step in a method for producing a biopolymer using a microorganism having the ability to produce the biopolymer.

In one aspect, the present invention provides an enzyme composition for biopolymer purification, comprising the protease of the present invention. The enzyme composition of the present invention may be a solid composition such as a powder or may be a liquid composition. The enzyme composition can be appropriately supplemented with a surfactant, a chelating agent, a water-soluble polymer, an alkali agent, an organic acid or a salt thereof, an additional enzyme other than the protease of the present invention, an enzyme stabilizer, an antioxidant, a solubilizer, a pH adjuster, a buffer, an antiseptic, a fragrance, and the like, in addition to the protease of the present invention.

The content of the protease of the present invention in the enzyme composition of the present invention is not particularly limited as long as the protease of the present invention in the amount exhibits activity. The content is preferably from 0.01 to 500 g, more preferably from 0.1 to 200 g, further preferably from 1 to 100 g, per kg of the enzyme composition.

The protease of the present invention is used singly or in the form of an enzyme composition containing this protease for purifying a biopolymer in a method for producing a biopolymer using a microorganism having the ability to produce the biopolymer. Thus, in another aspect, the present invention provides a method for producing a biopolymer using a microorganism having the ability to produce the biopolymer, comprising using the protease of the present invention in a purification step. In an alternative aspect, the present invention provides a method for purifying a biopolymer, comprising using the protease of the present invention in a method for producing a biopolymer using a microorganism having the ability to produce the biopolymer. Hereinafter, these methods are collectively referred to as the "method of the present invention".

The "microorganism having the ability to produce the biopolymer" in the method of the present invention is a microorganism having the ability to fermentatively produce the biopolymer and accumulate the biopolymer in a microbial cell. The microorganism is not particularly limited and may be a wild-type microorganism, or a mutant microorganism (mutant) having a mutation, such as insertion, substitution, or deletion, in a nucleotide sequence by various gene manipulations, or may be a recombinant microorganism provided with the ability to produce the desired biopolymer by a known artificial variation.

The method for producing a biopolymer using a microorganism having the ability to produce the biopolymer is a method of fermentatively producing a biopolymer using a microorganism having the ability to produce the biopolymer, and comprises steps which are generally used in the art, typically, a step of culturing the microorganism having the ability to produce the biopolymer, a biopolymer purification step, and a step of drying the biopolymer. The protease of the present invention is applied to the biopolymer purification step.

The "biopolymer" to be produced by the method for producing a biopolymer using a microorganism having the ability to produce the biopolymer as the method of the present invention refers to a polymer which is fermentatively produced by the microorganism and accumulated in a microbial cell, among polymers produced with biomasses as starting materials. Examples of the biopolymer include polyhydroxyalkanoate (PHA).

PHA is polyester formed from hydroxyalkanoic acid as a monomer unit, and is produced by a microorganism having the ability to produce PHA with a carbon source such as sugars, alcohols, fatty acid, or fat/oil and accumulated in a microbial cell of the microorganism. Examples of the hydroxyalkanoic acid include (R)-3-hydroxybutyric acid (3HB), 4-hydroxybutyric acid (4HB), 3-hydroxypropionic acid (3HP), (R)-3-hydroxypentanoic acid ((R)-3-hydroxyvaleric acid: 3HV), (R)-3-hydroxyhexanoic acid (3HHx), (R)-3-hydroxyoctanoic acid (3HO), (R)-3-hydroxydecanoic acid (3HD), (R)-3-hydroxydodecanoic acid (3HDD), and (R)-3-hydroxytetradecanoic acid (3HTD). PHA may be a homopolymer composed of one monomer unit or may be a copolymer composed of two or more monomer units. Examples of PHA include poly[(R)-3-hydroxybutyric acid] (P(3HB)), poly[(R)-3-hydroxybutyric acid-co-(R)-3-hydroxyvaleric acid](P(3HB-co-3HV)), poly[(R)-3-hydroxybutyric acid-co-(R)-4-hydroxybutyric acid](P(3HB-co-4HB)), poly[(R)-3-hydroxybutyric acid-co-(R)-3-hydroxyhexanoic acid](P(3HB-co-3HHx)), and poly[(R)-3-hydroxybutyric acid-co-(R)-3-hydroxydecanoic](P(3HB-co-3HD)). The compositional ratio of the monomer units in copolymerization is not particularly limited, and PHA having various compositional ratios can be obtained depending on the type of the microorganism having the ability to produce PHA, the type of the carbon source, difference in culture method, and so on.

The microorganism having the ability to produce PHA may be a wild-type microorganism intrinsically having the ability to produce PHA, may be a mutant microorganism having the altered ability to produce PHA by, for example, a variation in a gene encoding a PHA polymerizing enzyme, or may be a recombinant microorganism provided with the ability to produce PHA by the introduction of a gene encoding a PHA polymerizing enzyme from the outside. Examples of the microorganism include microorganisms belonging to the genus *Aeromonas,* the genus *Bacillus,* the genus *Cupriavidus,* the genus *Escherichia,* and the genus *Pseudomonas.* Among them, a microorganism belonging to the genus *Aeromonas,* the genus *Cupriavidus,* or the genus *Escherichia* is preferred, and a microorganism belonging to the genus *Cupriavidus* is more preferred, from the viewpoint of PHA productivity. Examples of the microorganism belonging to the genus *Cupriavidus* include *Cupriavidus necator* (formerly *Ralstonia eutropha*) and *Cupriavidus metallidurans.* In particular, *Cupriavidus necator* is preferred.

The step of culturing the microorganism having the ability to produce the biopolymer is a step of fermentatively producing the biopolymer using the microorganism. The step can be performed under general conditions which are adopted for biopolymer production using the microorganism having the ability to produce the biopolymer. For example, the culture medium for the culture may be a synthetic culture medium or a natural culture medium as long as the culture medium contains nutrients, such as a carbon source, a nitrogen source, and inorganic salts, necessary for biopolymer production using the microorganism having the ability to produce the biopolymer.

The carbon source may not be limited as long as the carbon source can be assimilated by the microorganism having the ability to produce the biopolymer. Examples thereof include sugars such as glucose, glycerol, fructose, sucrose, maltose, mannose, galactose, starch hydrolysates, and molasses; alcohols such as ethanol; fatty acids such as dodecanoic acid (lauric acid), tridecanoic acid, tetradecanoic acid (myristic acid), pentadecanoic acid (pentadecylic acid), hexadecanoic acid (palmitic acid), hexadecenoic acid, heptadecanoic acid (margaric acid), octadecanoic acid (stearic acid), octadecenoic acid, octadecadienoic acid, octadecatrienoic acid, nonadecanoic acid, eicosanoic acid, eicosadienoic acid, eicosatrienoic acid, and eicosatetraenoic acid; and fat/oil such as coconut oil, palm oil, palm kernel oil, olive oil, rapeseed oil, rice bran oil, soybean oil, castor oil, and mahua oil. One of these carbon sources can be used singly, or two or more thereof can be used as a mixture. The carbon source(s) can be added to the culture medium by an arbitrary method such as addition in one portion, addition in divided portions, or continuous addition.

Examples of the nitrogen source include ammonia, ammonium salts such as ammonium sulfate, nitrogen compounds such as amine, and natural nitrogen sources such as peptone and soybean hydrolysates.

Examples of the inorganic salts include disodium hydrogen phosphate, potassium dihydrogen phosphate, magnesium sulfate heptahydrate, iron(III) chloride, calcium chloride, cobalt chloride, copper sulfate, and nickel chloride.

The culture medium may be further supplemented with vitamins, if necessary. Examples of the vitamins include biotin, thiamine (vitamin B1), pyridoxin (vitamin B6), pantothenic acid, inositol, and nicotinic acid.

Aerobic conditions are preferred for the culture, and a general method such as aeration stirring culture or shake culture can be applied thereto. An arbitrary culture format can be selected from batch culture, semi-batch culture, continuous culture, and the like. The culture temperature is preferably from 10 to 50°C, more preferably from 20 to 42°C, further more preferably from 25 to 35°C. The initial pH (30°C) of the culture medium is preferably from 6 to 9, more preferably from 7 to 8. The culture time is preferably from 24 to 200 hours, more preferably from 50 to 100 hours.

After culture, the microorganism having the ability to produce the biopolymer in the cultures contains a biopolymer produced in a microbial cell. The cultures are subjected to a biopolymer purification step, and the microorganism having the ability to produce the biopolymer may be inactivated, if necessary, before this step. The inactivation of the microorganism having the ability to produce the biopolymer can be performed, for example, by heat-treating the microorganism at approximately 50 to 80°C for approximately 10 to 60 minutes. Furthermore, culture medium components may be removed, if necessary, before the biopolymer purification step to recover the microorganism having the ability to produce the biopolymer.

The biopolymer purification step is a step of degrading microbial cell-derived components, other than the biopolymer, of the microorganism having the ability to produce the biopolymer and containing the biopolymer (hereinafter, referred to as the "biopolymer-containing microorganism"), and separating the biopolymer from the biopolymer-containing microorganism to enhance the purity of the biopolymer. The step is performed using the protease of the present invention. The microbial cell-derived components other than the biopolymer are cell walls, proteins, sugars, nucleic acids, lipids, and the like derived from the microbial cell of the biopolymer-containing microorganism, except for the biopolymer, and are impurities, the contamination of which into the biopolymer is desired to be reduced.

In a preferable embodiment, the protease of the present invention is allowed to act on the biopolymer-containing microorganism or disrupted microbial cells thereof. Any approach of allowing the protease of the present invention to act on the biopolymer-containing microorganism or the disrupted microbial cells thereof may be used as long as the approach is known in the art. The protease of the present invention is preferably added to a suspension containing the biopolymer-containing microorganism or the disrupted microbial cells thereof. Conditions for the reaction of the biopolymer-containing microorganism or the disrupted microbial cells thereof with the protease of the present invention are not particularly limited as long as the conditions do not inactivate the protease of the present invention. Proper reaction conditions can be appropriately determined by those skilled in the art depending on the type, shape, and amount of the microorganism having the ability to produce the biopolymer, the type, shape, and amount of the biopolymer, and the type and amount of the protease of the present invention, and so on. Hereinafter, examples of the reaction conditions in the method of the present invention will be described.

The amount of the protease of the present invention used in the reaction is appropriately determined depending on the type, shape, and amount of the microorganism having the ability to produce the biopolymer, and so on. The amount of the protease of the present invention used is, for example, preferably from 0.1 to 10% by mass, more preferably from 0.1 to 5% by mass, further more preferably from 0.1 to 3% by mass, per 100% by mass of the microorganism having the ability to produce the biopolymer or the disrupted microbial cells thereof in terms of a dry mass.

The pH condition of the reaction is preferably pH 8 or higher, more preferably pH 9 or higher, and preferably pH 13 or lower, more preferably pH 12 or lower, from the viewpoint of improvement in biopolymer purification efficiency. The pH is preferably from pH 8 to 13, more preferably from pH 9 to 12, further more preferably pH 10. The pH can be adjusted by the addition of an appropriate acid or base.

The temperature condition of the reaction is preferably from 30 to 80°C, more preferably from 40 to 70°C, further more preferably from 40 to 60°C, from the viewpoint of improvement in biopolymer purification efficiency.

The reaction time is preferably from 30 minutes to 24 hours, more preferably from 1 to 12 hours, further more preferably from 1 to 5 hours, from the viewpoint of improvement in biopolymer purification efficiency.

The reaction is preferably performed in the presence of a surfactant from the viewpoint of improvement in biopolymer purification efficiency. Examples of the surfactant can include one or combinations of anionic surfactants, nonionic surfactants, amphoteric surfactants, and cationic surfactants. An anionic surfactant is preferred. Examples of the anionic surfactant can include sulfuric acid ester salts of alcohols having 10 to 18 carbon atoms, sulfuric acid ester salts of alkoxylates of alcohols having 8 to 20 carbon atoms, alkylbenzenesulfonates, paraffin sulfonate, α-olefin sulfonate, internal olefin sulfonate, α-sulfo fatty acid salts, α-sulfo fatty acid alkyl ester salts, and fatty acid salts. Particularly, one or more anionic surfactant selected from the group consisting of sulfuric acid esters of alcohols with an alkyl chain having 10 to 14 carbon atoms, and linear alkylbenzenesulfonates with an alkyl chain having 10 to 14 carbon atoms, are preferred. The counterion is preferably derived from an alkali metal salt or amines, particularly preferably sodium and/or potassium, monoethanolamine, or diethanolamine. Particularly, sodium dodecyl sulfate is preferred.

In the biopolymer purification step, other treatments such as mechanical treatment, heat treatment, alkali treatment, surfactant treatment, or oxidizing agent treatment may be performed in addition to the treatment with the protease of the present invention described above. These other treatments can be performed under general conditions which are adopted for biopolymer production using the microorganism having the ability to produce the biopolymer. The mechanical treatment means microbial cell disruption treatment under ultrasonic wave, pressure, or the like. Examples of the alkali include sodium hydroxide, sodium sesquicarbonate, and sodium bicarbonate. Examples of the surfactant can include one or combinations of anionic surfactants, nonionic surfactants, amphoteric surfactants, and cationic surfactants. An anionic surfactant is preferred. Examples of the anionic surfactant can include sulfuric acid ester salts of alcohols having 10 to 18 carbon atoms, sulfuric acid ester salts of alkoxylates of alcohols having 8 to 20 carbon atoms, alkylbenzenesulfonates, paraffin sulfonate, α-olefin sulfonate, internal olefin sulfonate, α-sulfo fatty acid salts, α-sulfo fatty acid alkyl ester salts, and fatty acid salts. Particularly, one or more anionic surfactant selected from the group consisting of sulfuric acid esters of alcohols with an alkyl chain having 10 to 14 carbon atoms, and linear alkylbenzenesulfonates with an alkyl chain having 10 to 14 carbon atoms, are preferred. The counterion is preferably an alkali metal salt or amines, particularly preferably sodium and/or potassium, monoethanolamine, or diethanolamine. Particularly, sodium dodecyl sulfate is preferred. Examples of the oxidizing agent include hydrogen peroxide, percarbonate, perborate, persulfate, sodium peroxide, ozone, chlorite, chlorate, perchlorate, and perchloric acid. One or more of these other treatments can be performed simultaneously and parallelly with the treatment with the protease of the present invention, or these treatments can be performed each independently. In the biopolymer purification step, treatments with enzymes other than the protease of the present invention may be performed in addition to the treatment with the protease of the present invention described above. These treatments with enzymes other than the protease of the present invention can be performed under general conditions which are adopted for biopolymer production using the microorganism having the ability to produce the biopolymer. The enzymes other than the protease of the present invention may not be limited as long as the enzymes are capable of hydrolyzing microbial cell-derived components other than the biopolymer. Examples thereof include lysozyme, muramidase, glycosidase, cellulase, nuclease, lipase, amylase, cutinase, laccase, and proteases other than the protease of the present invention. One or more of these treatments with the enzymes other than the protease of the present invention can be performed simultaneously and parallelly with the treatment with the protease of the present invention, or these treatments can be performed each independently.

Solid matter of the reaction solution obtained after the biopolymer purification step contains the biopolymer. Accordingly, the solid matter of the reaction solution is dried by the step of drying the biopolymer to recover the biopolymer. The step can be performed under general conditions which are adopted for biopolymer production using the microorganism having the ability to produce the biopolymer. The step can be performed, for example, by subjecting the reaction solution to solid-liquid separation by centrifugation or the like to give solid matter, washing the solid matter, if necessary, and then drying the solid matter by spray drying, evaporation to dryness, freeze drying, or the like. A powder of the biopolymer can be obtained by the step.

The biopolymer thus produced, as compared with biopolymers produced by conventional methods, has a much higher purity because contamination by residues, which are microbial cell-derived components of the microorganism having the ability to produce the biopolymer, is reduced. The purity of the biopolymer can be measured, for example, by using the ratio of the dry weight of residues contaminating a powder of the biopolymer to the weight of the powder of the biopolymer as an indicator in accordance with a method shown in Example 1(4) mentioned later. The dry weight of residues contaminating the powder of the biopolymer can be measured by dissolving the biopolymer by the addition of chloroform to the powder of the biopolymer, subsequently removing chloroform, and drying remaining residues. A biopolymer powder having a lower ratio of the dry weight of residues can be confirmed to have a higher biopolymer purity. The ratio of the dry weight of residues in the biopolymer produced by the method of the present invention can be preferably 20% or less, more preferably 18% or less.

The following compositions, production methods, applications, or methods are further disclosed as exemplary embodiments of the present invention in the present specification. However, the present invention is not limited to these embodiments.

[1] A method for producing a biopolymer using a microorganism having the ability to produce the biopolymer, comprising using at least one protease selected from the group consisting of the following a) and b) in a biopolymer purification step:
   a) a protease consisting of an amino acid sequence having at least 90% identity to SEQ ID NO: 1 or 4, and
   b) a protease consisting of an amino acid sequence having at least 90% identity to SEQ ID NO: 5 or 8.
[2] A method for purifying a biopolymer, comprising using at least one protease selected from the group consisting of the above a) and b) in a method for producing a biopolymer using a microorganism having the ability to produce the biopolymer.
[3] The method according to [1] or [2], wherein the protease is at least one protease selected from the group consisting of the following a') and b'):
   a') a protease which consists of an amino acid sequence having at least 90% identity to SEQ ID NO: 1 or 4 and has at least one amino acid residue selected from the group consisting of valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1, and
   b') a protease which consists of an amino acid sequence having at least 90% identity to SEQ ID NO: 5 or 8 and has at least one amino acid residue selected from the group consisting of glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.
[4] The method according to any one of [1] to [3], wherein the protease consists of an amino acid sequence having at least 90% identity to SEQ ID NO: 1 or 4 and has valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1.
[5] The method according to any one of [1] to [3], wherein the protease consists of an amino acid sequence having at least 90% identity to SEQ ID NO: 5 or 8 and has glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.
[6] The method according to any one of [1] to [5], wherein the protease is allowed to act on a microorganism containing the biopolymer or disrupted microbial cells thereof.
[7] The method according to any one of [1] to [6], wherein the protease is allowed to act on a microorganism containing the biopolymer or disrupted microbial cells thereof under a condition of pH 8 or higher, preferably pH 9 or higher, and pH 13 or lower, preferably pH 12 or lower, or from pH 8 to 13, preferably from pH 9 to 12, more preferably pH 10.
[8] The method according to any one of [1] to [7], wherein the protease is allowed to act on a microorganism containing the biopolymer or disrupted microbial cells thereof in the presence of a surfactant, preferably in the presence of sodium dodecyl sulfate.
[9] The method according to any one of [1] to [8], wherein the biopolymer is polyhydroxyalkanoate (PHA).
[10] The method according to any one of [1] to [9], wherein the microorganism having the ability to produce the biopolymer is a microorganism belonging to the genus *Cupriavidus,* preferably *Cupriavidus necator.*
[11] The method according to any one of [1] to [10], wherein the method for producing a biopolymer using a microorganism having the ability to produce the biopolymer comprises a step of culturing the microorganism having the ability to produce the biopolymer, a biopolymer purification step, and a step of drying the biopolymer.
[12] An enzyme composition for biopolymer purification, comprising at least one protease selected from the group consisting of the above a) and b).
[13] The enzyme composition according to [12], wherein the protease is at least one protease selected from the group consisting of the above a') and b').
[14] The enzyme composition according to [12] or [13], wherein the protease consists of an amino acid sequence having at least 90% identity to SEQ ID NO: 1 or 4 and has valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1.
[15] The enzyme composition according to [12] or [13], wherein the protease consists of an amino acid sequence having at least 90% identity to SEQ ID NO: 5 or 8 and has glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.
[16] The enzyme composition according to any one of [12] to [15], wherein the biopolymer is PHA.
[17] The enzyme composition according to any one of [12] to [16], wherein the enzyme composition is used in a method for producing a biopolymer using a microorganism having the ability to produce the biopolymer.
[18] Use of at least one protease selected from the group consisting of the above a) and b) for the production of an enzyme composition for biopolymer purification.
[19] Use of at least one protease selected from the group consisting of the above a) and b) as an enzyme for biopolymer purification.
[20] The use according to [18] or [19], wherein the protease is at least one protease selected from the group consisting of the above a') and b').
[21] The use according to any one of [18] to [20], wherein the protease consists of an amino acid sequence having at least 90% identity to SEQ ID NO: 1 or 4 and has valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1.
[22] The use according to any one of [18] to [20], wherein the protease consists of an amino acid sequence having at least 90% identity to SEQ ID NO: 5 or 8 and has glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.
[23] The use according to any one of [18] to [22], wherein the biopolymer is PHA.
[24] A method for using at least one protease selected from the group consisting of the above a) and b) as an enzyme for biopolymer purification.
[25] The method according to [24], wherein the protease is at least one protease selected from the group consisting of the above a') and b').
[26] The method according to [24] or [25], wherein the protease consists of an amino acid sequence having at least 90% identity to SEQ ID NO: 1 or 4 and has valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1.
[27] The method according to [24] or [25], wherein the protease consists of an amino acid sequence having at least 90% identity to SEQ ID NO: 5 or 8 and has glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.
[28] The method according to any one of [24] to [27], wherein the biopolymer is PHA.

### Examples

Hereinafter, the present invention will be described in more detail with reference to Examples. However, the technical scope of the present invention is not limited to the following Examples.

### Example 1

### (1) Construction of protease expression plasmid

KP43 was expressed using the plasmid pHY-KP43 described in JP-A-2021-97605. pHY-KP43 has a gene encoding KP43 preproprotein (polynucleotide of SEQ ID NO: 3, which encodes the amino acid sequence of SEQ ID NO: 2). The amino acid sequence of a mature form of KP43 is shown in SEQ ID NO: 1.

Mutations were introduced into the protease by use of the site-directed mutagenesis method based on PCR using a pair of complementary primers (Zheng, Lei, Ulrich Baumann, and Jean-Louis Reymond. Nucleic Acids Research 32. 14 (2004): e115-e115).

### (2) Preparation of protease solution

The protease expression plasmid was introduced into a *Bacillus subtilis* strain by the protoplast technique, and the strain was cultured at 30°C for 3 days in 2 × L-maltose medium (2% tryptone, 1% yeast extracts, 1% NaCl, 7.5% maltose, 7.5 ppm manganese sulfate pentahydrate, 0.04% calcium chloride dihydrate, 15 ppm tetracycline; % denotes (w/v) %). Then, a culture supernatant containing the protease was recovered by centrifugation. The culture supernatant was buffer-replaced with 20 mM Tris-HCl (pH 7.5) containing 2 mM CaCl₂ using Amicon 10K to prepare a protease solution. Reagents were used as protease solutions for Alcalase (Sigma-Aldrich Co., LLC, 126741) and Esperase (Sigma-Aldrich Co., LLC, P5860).

The concentrations of the protease solutions were measured using DC Protein Assay Kit (Bio-Rad Laboratories, Inc.). BSA Standard Solution (FUJIFILM Wako Pure Chemical Corp.) was used as a standard solution for calculating the amounts of proteins.

### (3) Preparation of PHA-containing microbial cell

*Cupriavidus necator (Ralstonia eutropha)* NBRC 102504 strain was inoculated to an LB liquid medium and shakecultured at 30°C for 24 hours. 1 mL of this culture solution was inoculated to a 500 mL baffled flask supplemented with 100 mL of a culture medium for PHA production (1.1% disodium hydrogen phosphate dodecahydrate, 0.19% potassium dihydrogen phosphate, 1.29% ammonium sulfate, 0.1% magnesium sulfate heptahydrate, 0.5% 80 ppm iron(III) chloride hexahydrate, 50 ppm calcium chloride dihydrate, 1 ppm cobalt chloride hexahydrate, 0.8 ppm copper sulfate pentahydrate, 0.6 ppm nickel chloride hexahydrate, 1% coconut oil), and shakecultured at 30°C for 24 hours. 24 hours later, 9 g of coconut oil was added thereto, followed by further culture for 48 hours. The microbial cell culture solution was heated at 60°C for 20 minutes to inactivate the microbial cells. The inactivated culture solution was dispensed in 15 mL each to 50 mL tubes and centrifuged at 8,000 rpm for 10 minutes to give PHA-containing microbial cell pellets.

### (4) PHA recovery using protease - 1

The pellets obtained in (3) were suspended by the addition of a 1 mM aqueous sodium hydroxide solution (pH 11) containing 0.2% by weight of sodium dodecyl sulfate in 15 mL per tube containing the pellets, and the tube was incubated at 50°C for 1 hour. The microbial cells were disrupted (output: high, 10 cycles each involving 30-sec homogenization and 30-sec rest) using an ultrasonic disruptor Bioruptor II (Sonicbio Co., Ltd.). 15 mL of distilled water was added to the disrupted microbial cells in each tube, and the tube was centrifuged at 10,000 rpm for 10 minutes, followed by the discarding of 15 mL of a supernatant. 15 mL of a 1 mM aqueous sodium hydroxide solution (pH 11) was added to each tube, and the tube was centrifuged at 10,000 rpm for 20 minutes, followed by the removal of a supernatant. Again, 15 mL of a 1 mM aqueous sodium hydroxide solution (pH 11) was added to each tube, and the tube was centrifuged at 10,000 rpm for 20 minutes, followed by the removal of a supernatant. The pellets were suspended in 15 mL of an aqueous solution of sodium dodecyl sulfate with a final concentration of 0.2% by weight, the pH of which was adjusted to 10 by the addition of sodium hydroxide. 200 µg of each protease was added thereto and thoroughly mixed. After reaction at 50°C for 2 hours with shaking and stirring, the reaction solution was centrifuged at 10,000 rpm for 20 minutes, and the obtained pellets were washed three times with a 1 mM aqueous sodium hydroxide solution (pH 11). The obtained pellets were freeze-dried to give a powder containing PHA.

The dry powder obtained above was placed in a glass test tube, the weight of which was already measured, and the weight of the resultant was measured. 2 mL of chloroform was added thereto, and the mixture was thoroughly stirred. The test tube was centrifuged, and chloroform was removed. Again, 1 mL of chloroform was added thereto, and the mixture was thoroughly stirred, followed by centrifugation and the removal of chloroform. Again, 2 mL of chloroform was added thereto, and the mixture was thoroughly stirred, followed by centrifugation and the removal of chloroform. Residues remaining in the test tube were dried, and the weight thereof was then measured. The ratio of the weight of the residues to the powder weight before PHA dissolution was determined (Table 1). The residues are microbial cell-derived impurities because PHA is soluble in chloroform.

Wild-type KP43 and KP43 mutant S16V + T65P + N83A + V273I + T359S + S387A + A132T + N166G + G167V + Y195Q + A294T + D369N had a significant effect of reducing the amount of residues, as compared with Alcalase and Esperase. Particularly, KP43 mutant S16V + T65P + N83A + V273I + T359S + S387A + A132T + N166G + G167V + Y195Q + A294T + D369N drastically decreased the amount of residues even when compared with wild-type KP43.

**[Table 1]**

| Enzyme | Residue (%) |
|---|---|
| Alcalase | 21.1 |
| Esperase | 23.0 |
| Wild-type KP43 | 20.0 |
| KP43 S16V + T65P + N83A + V2731 + T359S + S387A + A132T + N166G + G167V + Y195Q + A294T + D369N | 15.8 |

### (5) PHA recovery using protease - 2

PHA recovery was performed in the same manner as in (4) except that no ultrasonic homogenization was performed. The amount of residues was measured after each protease treatment (Table 2).

KP43 mutant S16V + T65P + N83A + V273I + T359S + S387A + A132T + N166G + G167V + Y195Q + A294T + D369N drastically decreased the amount of residues as compared with wild-type KP43.

**[Table 2]**

| Enzyme | Residue (%) |
|---|---|
| No enzyme | 26.7 |
| Wild-type KP43 | 22.0 |
| KP43 S16V + T65P + N83A + V2731 + T359S + S387A + A132T + N166G + G167V + Y195Q + A294T + D369N | 12.8 |

### Example 2

### (1) Construction of protease expression plasmid

A K16 expression plasmid pHY-K16 was constructed by using a plasmid for the expression of VHH of SEQ ID NO: 26 containing a *Bacillus subtilis* spoVG gene-derived promoter described in WO 2021/153129 as a template and transferring artificially synthesized K16 gene (polynucleotide of SEQ ID NO: 7, which encodes K16 preproprotein of SEQ ID NO: 6) to full-length ORF including the VHH gene by seamless cloning. The amino acid sequence of a mature form of K16 is shown in SEQ ID NO: 5.

Mutations were introduced into the protease by use of the site-directed mutagenesis method based on PCR using a pair of complementary primers (Zheng, Lei, Ulrich Baumann, and Jean-Louis Reymond. Nucleic Acids Research 32. 14 (2004): e115-e115).

### (2) Preparation of protease solution

A protease solution was prepared in the same manner as in Example 1(2).

### (3) PHA recovery using protease - 3

PHA recovery was performed in the same manner as in Example 1(5), and the amount of residues was measured after each protease treatment. The ratio (%) of the weight of residues to the powder weight before PHA dissolution was calculated, and a difference from the ratio (%) of residues obtained using Alcalase was determined (Table 3). Positive values indicate a larger effect of reducing the amount of residues than that of Alcalase.

Wild-type K16 had a significant effect of reducing the amount of residues as compared with Alcalase. Furthermore, mutant S9E, mutant L256E, and mutant S9E + N253D + N255W + L256E had a significant effect of reducing the amount of residues as compared with wild-type K16.

**[Table 3]**

| Enzyme | Difference from Alcalase (Δ%) |
|---|---|
| Alcalase | 0.0 |
| Wild-type K16 | 2.8 |
| K16 S9E | 4.4 |
| K16 L256E | 5.6 |
| K16 S9E + N253D + N255W + L256E | 8.1 |

## Claims

1. A method for producing a biopolymer using a microorganism having the ability to produce the biopolymer, comprising using at least one protease selected from the group consisting of the following a) and b) in a biopolymer purification step:
a) a protease consisting of an amino acid sequence having at least 90% identity to SEQ ID NO: 1 or 4, and
b) a protease consisting of an amino acid sequence having at least 90% identity to SEQ ID NO: 5 or 8.

2. A method for purifying a biopolymer, comprising using at least one protease selected from the group consisting of the following a) and b) in a method for producing a biopolymer using a microorganism having the ability to produce the biopolymer:
a) a protease consisting of an amino acid sequence having at least 90% identity to SEQ ID NO: 1 or 4, and
b) a protease consisting of an amino acid sequence having at least 90% identity to SEQ ID NO: 5 or 8.

3. The method according to claim 1 or 2, wherein the protease is at least one protease selected from the group consisting of the following a') and b'):
a') a protease which consists of an amino acid sequence having at least 90% identity to SEQ ID NO: 1 or 4 and has at least one amino acid residue selected from the group consisting of valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1, and
b') a protease which consists of an amino acid sequence having at least 90% identity to SEQ ID NO: 5 or 8 and has at least one amino acid residue selected from the group consisting of glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.

4. The method according to any one of claims 1 to 3, wherein the protease is allowed to act on a microorganism containing the biopolymer or disrupted microbial cells thereof.

5. The method according to any one of claims 1 to 4, wherein the biopolymer is polyhydroxyalkanoate (PHA).

6. The method according to any one of claims 1 to 5, wherein the microorganism having the ability to produce the biopolymer is a microorganism belonging to the genus *Cupriavidus.*

7. An enzyme composition for biopolymer purification, comprising at least one protease selected from the group consisting of the following a) and b):
a) a protease consisting of an amino acid sequence having at least 90% identity to SEQ ID NO: 1 or 4, and
b) a protease consisting of an amino acid sequence having at least 90% identity to SEQ ID NO: 5 or 8.

8. The enzyme composition according to claim 7, wherein the protease is at least one protease selected from the group consisting of the following a') and b'):
a') a protease which consists of an amino acid sequence having at least 90% identity to SEQ ID NO: 1 or 4 and has at least one amino acid residue selected from the group consisting of valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1, and
b') a protease which consists of an amino acid sequence having at least 90% identity to SEQ ID NO: 5 or 8 and has at least one amino acid residue selected from the group consisting of glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.

9. The enzyme composition according to claim 7 or 8, wherein the biopolymer is PHA.

10. Use of at least one protease selected from the group consisting of the following a) and b) for the production of an enzyme composition for biopolymer purification:
a) a protease consisting of an amino acid sequence having at least 90% identity to SEQ ID NO: 1 or 4, and
b) a protease consisting of an amino acid sequence having at least 90% identity to SEQ ID NO: 5 or 8.

11. Use of at least one protease selected from the group consisting of the following a) and b) as an enzyme for biopolymer purification:
a) a protease consisting of an amino acid sequence having at least 90% identity to SEQ ID NO: 1 or 4, and
b) a protease consisting of an amino acid sequence having at least 90% identity to SEQ ID NO: 5 or 8.

12. The use according to claim 10 or 11, wherein the protease is at least one protease selected from the group consisting of the following a') and b'):
a) a protease which consists of an amino acid sequence having at least 90% identity to SEQ ID NO: 1 or 4 and has at least one amino acid residue selected from the group consisting of valine at a position corresponding to position 16, proline at a position corresponding to position 65, alanine at a position corresponding to position 83, isoleucine at a position corresponding to position 273, serine at a position corresponding to position 359, alanine at a position corresponding to position 387, threonine at a position corresponding to position 132, glycine at a position corresponding to position 166, valine at a position corresponding to position 167, glutamine at a position corresponding to position 195, threonine at a position corresponding to position 294, and asparagine at a position corresponding to position 369 according to numbering based on SEQ ID NO: 1, and
b) a protease which consists of an amino acid sequence having at least 90% identity to SEQ ID NO: 5 or 8 and has at least one amino acid residue selected from the group consisting of glutamic acid at a position corresponding to position 9, aspartic acid at a position corresponding to position 253, tryptophan at a position corresponding to position 255, and glutamic acid at a position corresponding to position 256 according to numbering based on SEQ ID NO: 5.

13. The use according to any one of claims 10 to 12, wherein the biopolymer is PHA.
